# EUROPEAN PATENT SPECIFICATION

(11) **EP 3 534 835 B1**
(45) Date of publication and mention of the grant of the patent: **14.05.2025**
(21) Application number: 17895433.5
(22) Date of filing: 03.11.2017
(51) Int. Cl.: A61F 2/02

(54) **STACKED TISSUE ENCAPSULATION DEVICE SYSTEMS**
GESTAPELTE GEWEBEVERKAPSELUNGSVORRICHTUNGSSYSTEME
SYSTÈMES DE DISPOSITIFS À ENCAPSULATION TISSULAIRE EMPILÉS

(30) Priority: 03.11.2016 US 201661417017 P
(43) Date of publication of application: 11.09.2019
(73) Proprietor: Arizona Board of Regents on behalf of the University of Arizona, Tucson, AZ 85721 (US)
(72) Inventor: PAPAS, Klearchos K., Tucson, AZ 85724 (US)
(74) Representative: J A Kemp LLP
(86) International application number: PCT/US2017/060034
(87) International publication number: WO 2018/144098

(56) References cited:
- WO-A2-2008/100559
- JP-A- H06 205 665
- US-A- 6 060 640
- US-A- 6 143 293
- US-A1- 2007 061 015
- US-A1- 2010 082 114
- US-A1- 2010 082 114
- US-A1- 2010 255 059
- US-A1- 2011 092 949
- US-A1- 2014 257 515
- US-A1- 2014 257 515
- US-A1- 2014 257 515
- US-A1- 2015 112 247
- US-A1- 2015 320 836
- US-A1- 2016 184 569
- US-B1- 6 562 616

## Description

### FIELD OF THE INVENTION

The present invention relates to encapsulation devices for cells (such as but not limited to islet cells or stem cell derived beta cells or the like, e.g., for regulating blood glucose, or other cells or spheroids that can produce and release a therapeutic agent that is useful in the body), more particularly systems of encapsulation devices comprising two, three, or a plurality of encapsulation devices stacked together with the ability to form blood vessels around and in between them.

### BACKGROUND OF THE INVENTION

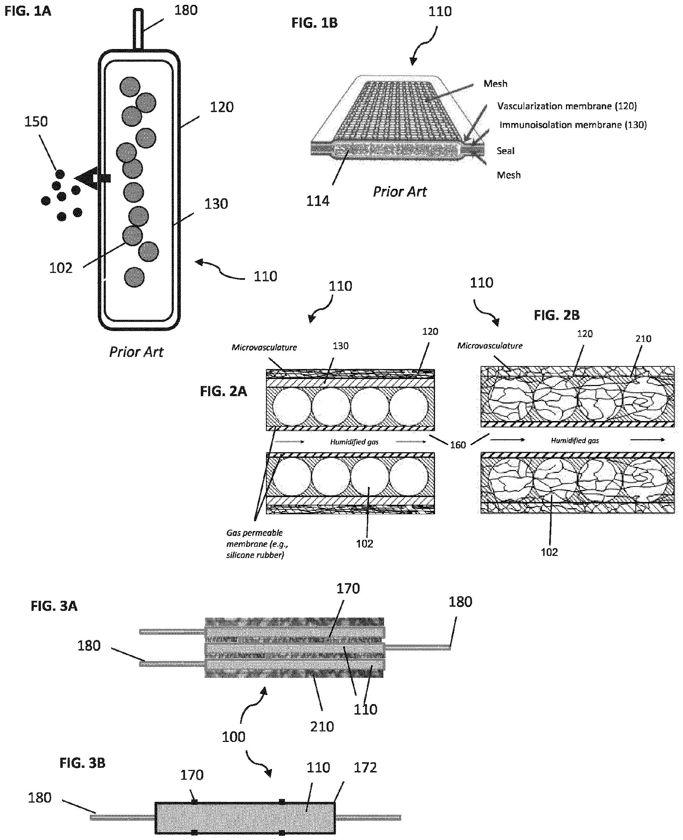

Immunoisolation, or implantation of tissues in a membrane-enclosed device (encapsulation device), are approaches aimed at allowing maintenance of cells in an environment where they are segregated form the host tissues. A non-limiting example of an encapsulation device is shown in FIG. 1A and FIG. 1B.

It was surprisingly discovered that a system comprising stacked encapsulation devices (e.g., 4 devices) could be implanted and vascularization occurred in between the stacked devices. The system (stacked encapsulation devices) was easily explanted from animals without major bleeding, even with vasculature around and in between the stacked encapsulation devices.

The vascularization around and in between the system (stacked encapsulation devices) can help the delivery of oxygen and nutrients from the blood supply to the encapsulated cells while enabling the reduction in the required device footprint (the system with the stacked devices would take up less room than having each encapsulation device implanted separately next to each other) and may reduce or eliminate the need for exogenous oxygen delivery (typically, one of ordinary skill in the art would expect that if you are packing cells at a high density, exogenous oxygen delivery would be needed). US 2014/257515 A1 relates to a 3-dimensional large capacity cell encapsulation device assembly.

### SUMMARY OF THE INVENTION

The invention is defined in claim 1. Further aspects and preferred embodiments are defined in the dependent claims. Any aspects, embodiments and examples of the present disclosure which do not fall under the scope of the appended claims do not form part of the invention and are merely provided for illustrative purposes. The present invention features systems comprising two or more encapsulation devices for cells that are stacked to create a thicker unit, e.g., two devices stacked, three devices stacked, four devices stacked, five devices stacked, six devices stacked, etc. Vascularization occurs within and around the system, which can help the delivery of oxygen and nutrients from the blood supply to the encapsulated cells while enabling the reduction in the required device footprint.

The connecting component does not cover the entire periphery so as to allow vasculature to grow between the devices.

The systems of the present invention may be used in conjunction with other therapies such as an artificial pancreas (e.g., a glucose sensor and an insulin infusion pump or a combination of the two and a control algorithm, etc.).

In some embodiments, cells are not present in the system or device upon implantation. In some embodiments, sensors may detect when oxygen is present in the encapsulation devices (e.g., vascularization has occurred), and cells may be subsequently injected into the device. In some embodiments, a user can determine how much oxygen is present in the system (or device) and determine how many cells to implant based on the oxygen level.

Implantation may be at any appropriate site, including but not limited to an arm location, a leg location, a torso location, etc.

The present invention features a system comprising at least a first encapsulation device stacked on a second encapsulation device and connected by a connecting component (e.g., a suture, etc.). The connecting component allows vasculature to grow between the two encapsulation devices (e.g., the two devices are not sealed to the point that they don't allow for cell penetration between them, e.g., as opposed to a full seal that closes off space between the devices so that vasculature cannot grow). The connecting component is provided to space the devices apart. Wherein the first encapsulation device and the second encapsulation device each comprise a lumen for holding cells and a vascularization membrane (e.g., surrounding the cells). Vasculature surrounds at least a portion of the system and is disposed in at least a portion of a space between the first encapsulation device and the second encapsulation device.

The first encapsulation device is separated from the second encapsulation device a particular distance (e.g., to allow vasculature growth). In some embodiments, the first encapsulation device further comprises an immunoisolation membrane in between the lumen and the vascularization membrane. In some embodiments, the second encapsulation device further comprises an immunoisolation membrane in between the lumen and the vascularization membrane. In some embodiments, the first encapsulation device and the second encapsulation device each comprise two lumens separated by a gas channel. In some embodiments, the gas channel of the first encapsulation device is fluidly connected to the gas channel of the second encapsulation device.

In some embodiments, the system further comprises cells disposed in the lumen of the first encapsulation device and the second encapsulation device. In some embodiments, the cells are for regulating blood glucose. In some embodiments, the cells for regulating blood glucose comprise islet cells or stem cell derived beta cells. In some embodiments, the cells comprise cells or spheroids that can produce and release a therapeutic agent.

In some embodiments, the lumen of the first encapsulation device or the lumen of the second encapsulation device comprises at least a small number of therapeutic cells that can survive and release pro-angiogenic factors that will enhance formation of blood vessels. In some embodiments, the system comprises a gel disposed between the first encapsulation device and the second encapsulation device. In some embodiments, pro-angiogenic factors are embedded in the gel. In some embodiments, the pro-angiogenic factors can be slowly released to enhance vascularization.

In some embodiments, the system is operatively connected to an oxygen generator, e.g., an implantable oxygen generator, a wearable oxygen generator, etc.

In some embodiments, the system further comprises one or more additional encapsulation devices. For example, in some embodiments, the system comprises three encapsulation devices stacked together and connected by a connecting component. In some embodiments, the system comprises four encapsulation devices stacked together and connected by a connecting component. In some embodiments, the system comprises five encapsulation devices stacked together and connected by a connecting component. In some embodiments, the system comprises six encapsulation devices stacked together and connected by a connecting component. In some embodiments, the system comprises seven encapsulation devices stacked together and connected by a connecting component.

According to the present invention, the connecting component separates the encapsulation devices to allow vasculature to grow. In some embodiments, the system further comprises an oxygen sensor. In some embodiments, the oxygen sensor is disposed on an outer surface of the system. In some embodiments, the oxygen sensor is disposed in the system. In some embodiments, the oxygen sensor is disposed in a gas channel of the system. In some embodiments, the system further comprises a glucose sensor. In some embodiments, the system is implanted into a subject without cells in the lumens and cells are inserted into the lumens after implantation. In some embodiments, the encapsulation devices comprise loading ports for introducing cells to the lumens.

The present invention also features a system comprising at least a first encapsulation device stacked on a second encapsulation device and connected by a connecting component, and a third encapsulation device stacked on the second encapsulation device and connected by a connecting component, wherein the encapsulation devices each comprise a lumen for holding cells surrounded by a vascularization membrane. Or, the present invention also features a system comprising at least a first encapsulation device stacked on a second encapsulation device and connected by a connecting component, and a third encapsulation device stacked on the second encapsulation device and connected by a connecting component, and a fourth encapsulation device stacked on the third encapsulation device, wherein the encapsulation devices each comprise a lumen for holding cells surrounded by a vascularization membrane.

In some embodiments, vasculature surrounds at least a portion of the system and is disposed in at least a portion of a space between the first encapsulation device and the second encapsulation device and in at least a portion of a space between the second encapsulation device and the third encapsulation device and in at least a portion of a space between the third encapsulation device and the fourth encapsulation device. In some embodiments, vasculature surrounds at least a portion of the system and is disposed in at least a portion of a space between the first encapsulation device and the second encapsulation device and in at least a portion of the space between the second encapsulation device and the third encapsulation device.

In some embodiments, the gas channel of the first encapsulation device is fluidly connected to the gas channel of the second encapsulation device, which is fluidly connected to the gas channel of the third encapsulation device. In some embodiments, the gas channel of the first encapsulation device is fluidly connected to the gas channel of the second encapsulation device, which is fluidly connected to the gas channel of the third encapsulation device, which is fluidly connected to the gas channel of the fourth encapsulation device.

In some embodiments, the connecting component comprises a suture. In some embodiments, the encapsulation devices each further comprise an immunoisolation membrane in between the lumen and the vascularization membrane. In some embodiments, the encapsulation devices each comprise two lumens separated by a gas channel.

In some embodiments, the system further comprises cells disposed in the lumen of each encapsulation device. In some embodiments, the cells are for regulating blood glucose. In some embodiments, the cells for regulating blood glucose comprise islet cells or stem cell derived beta cells. In some embodiments, the cells comprise cells or spheroids that can produce and release a therapeutic agent. In some embodiments, the lumens of the encapsulation devices comprise at least a small number of therapeutic cells that can survive and release pro-angiogenic factors that will enhance formation of blood vessels.

In some embodiments, the system comprises a gel disposed between the encapsulation devices. In some embodiments, pro-angiogenic factors are embedded in the gel. In some embodiments, the pro-angiogenic factors can be slowly released to enhance vascularization.

In some embodiments, the system is operatively connected to an oxygen generator. In some embodiments, the oxygen generator is an implantable oxygen generator. In some embodiments, the oxygen generator is a wearable oxygen generator.

The connecting component separates the encapsulation devices to allow vasculature to grow.

In some embodiments, the system comprises an oxygen sensor. In some embodiments, the system comprises a glucose sensor. In some embodiments, the system is implanted into a subject without cells in the lumens and cells are inserted into the lumens after implantation.

The disclosures of the following U.S. Patents are referred to: U.S. Pat. No. 5,713,888; U.S. Pat. App. No. 2003/0087427. Additional advantages and aspects of the present invention are apparent in the following detailed description and claims.

### BRIEF DESCRIPTION OF THE DRAWINGS

The features and advantages of the present invention will become apparent from a consideration of the following detailed description presented in connection with the accompanying drawings in which:
FIG. 1A shows an example of a single-chamber encapsulation device for holding cells or tissues. The device comprises a port to access the lumen for loading the cells or tissue.
FIG. 1B shows a cross-sectional view of the device of FIG. 1A The cells are encapsulated in a two-layer membrane envelope formed using a mesh insert. The device comprises a vascularization membrane and an immunoisolation membrane. The present invention is not limited to devices that utilize an immunoisolation membrane: in some embodiments, the device only comprises the vascularization membrane.
FIG. 2A shows a detailed view of an encapsulation device with an immunoisolation membrane. The device has two lumens or chambers with cells separated by a gas chamber.
FIG. 2B shows a detailed view of an encapsulation device without the immunoisolation membrane. The device has two lumens or chambers with cells separated by a gas chamber.
FIG. 3A shows a side view of a schematic of a system of the present invention comprising three encapsulation devices.
FIG. 3B shows a top view of the system of FIG. 3A.
FIG. 4 shows a schematic view of a system comprising four encapsulation devices stacked atop each other, each device with two lumens separated by a gas channel.
FIG. 5A shows a schematic view of a system connected to an implantable oxygen generator.
FIG. 5B shows a schematic view of a system connected to a wearable oxygen generator.
FIG. 6A shows an H&E stain of the system of Example 1 (three 40 µL devices stacked).
FIG. 6B shows an H&E stain of the system of Example 1 (three 40 µL devices stacked).
FIG. 6C shows an H&E stain of the system of Example 1 (three 40 µL devices stacked).
FIG. 7A shows an H&E stain of the 4.5 µL chamber system of Example 2.
FIG. 7B shows an H&E stain of the 4.5 µL chamber system of Example 2.
FIG. 7C shows an H&E stain of the 20 µL chamber system of Example 2.
FIG. 7D shows an H&E stain of the 20 µL chamber system of Example 2.

### DETAILED DESCRIPTION OF THE INVENTION

### Encapsulation Devices

Encapsulation devices are devices for holding cells or tissues. The encapsulation device (110) shown in FIG. 1A is a single-chamber encapsulation device. The device (100) comprises an inner lumen for holding the cells (102) or tissue and at least one vascularization membrane (120), which is impermeable to cells. In some embodiments, the device (100) further comprises an immunoisolation membrane (130). Non-cell factors or molecules (150) can escape the cell impermeable membrane. The device (110) also comprises a port (180) to access the lumen for loading the cells or tissue. FIG. 1B shows a cross-sectional view of an encapsulation device. The cells are encapsulated in a lumen (114) by a two-layer membrane envelope, a vascularization membrane (120) and an immunoisolation membrane (130). The device (110) also has structural support, e.g., mesh, seals, etc.

In some embodiments, the encapsulation devices (110) comprise a vascularization membrane (120) and immunoisolation membrane (130). In some embodiments, the encapsulation devices (110) comprise just the vascularization membrane (120). This allows blood vessels to grow within the transplanted tissue.

In the examples shown in FIG. 1A and FIG. 1B, the cells therein are about 5-15 µm in diameter. The outer membrane, the vascularization membrane (120), has a pore size from 5-10 µm. The vascularization membrane (120) is about 15 µm thick. The immunoisolation membrane (130) has a pore size of about 0.4 µm. The immunoisolation membrane (130) is about 30 µm thick. In some embodiments, the membranes (120, 130) are constructed from materials such as polytetraflouroethylene (PTFE) or other similar material. The present invention is not limited to the aforementioned pore sizes and thicknesses of the membranes used therein. The present invention is not limited to the aforementioned materials.

The encapsulation devices (110) may be constructed in various shapes and sizes and with various lumen volumes. For example, in some embodiments, the lumen has a volume of about 4.5 µl. In some embodiments, the lumen has a volume of 20 µl. In some embodiments, the lumen has a volume of 40 µl. In some embodiments, the device (110) is from 4 to 5 cm in length. In some embodiments, the device (110) is from 2 to 5 cm in length, e.g., 3 cm. In some embodiments, the device (110) is from 5 to 10 cm in length. The present invention is not limited to the aforementioned dimensions and lumen volumes. For example, in some embodiments, the lumen has a volume of about 100 µl. In some embodiments, the lumen has a volume of about 200 µl. In some embodiments, the lumen has a volume from 2 to 50 µl. In some embodiments, the lumen has a volume from 10 to 100 µl. In some embodiments, the lumen has a volume from 40 to 200 µl. In some embodiments, the lumen has a volume from 100 to 300 µl. In some embodiments, the lumen has a volume from 200 to 500 µl.

In some embodiments, within the encapsulation devices (110), there may be layers of cells or tissue, e.g., multiple lumens within the device (110). For example, an encapsulation device (110) may comprise two chambers or lumens. In some embodiments, the device comprises more than two chambers or lumens, e.g., 3 chambers or lumens, 4 chambers or lumens, 5 chambers or lumens, etc. FIG. 2A and FIG. 2B show examples an encapsulation with two lumens (two chambers) that are separated by a gas channel (160). FIG. 2A and FIG. 2B also show vascularizing membrane and microvasculature. The blood vessels embed into the vascularizing membrane.

In some embodiments, the chamber or lumen comprises a single layer of cells. In some embodiments, the chamber or lumen comprises two layers of cells. In some embodiments, the chamber comprises three or more layers of cells. In some embodiments, islet spheroids (about 150 um in size) are used (shown in FIG. 2A, FIG. 2B). In some embodiments, a dual layer of the islet spheroids is used (lumen thickness would be about 300 um in the chamber or in each chamber). In some embodiments, a third layer is supported depending on the metabolic activity and other characteristics of the spheroids/cells used. Note spheroids may not be touching each other in some configurations and the space between them may be 1 or 2 spheroids apart (e.g., 150 um, 300 um), or more or less.

### Systems with Stacked Encapsulation Devices

The present invention features a system (100) comprising two or more stacked encapsulation devices (110), e.g., a first encapsulation device and a second encapsulation device. The cells used in the various encapsulation devices of the system (100) may include but are not limited to islet cells or stem cell derived beta cells or the like, e.g., for regulating blood glucose, or other cells or spheroids that can produce and release a therapeutic agent that is useful in the body. The cells in the different encapsulation devices (110) may be the same, similar, or various different combinations of cells may be included within the encapsulation devices or throughout the stacked devices. For example, in a system (100) with two encapsulation devices (110), the cells in the first encapsulation device maybe the same as the cells in the second encapsulation device. Or, in some embodiments, the cells in the first encapsulation device maybe the different from the cells in the second encapsulation device.

In some embodiments, the system (100) comprises two encapsulation devices (110). In some embodiments, the system comprises three encapsulation devices. In some embodiments, the system comprises four encapsulation devices. In some embodiments, the system comprises five encapsulation devices. In some embodiments, the system comprises six encapsulation devices. In some embodiments, the system comprises more than six encapsulation devices, e.g., seven devices, eight devices, nine devices, ten devices, more than ten devices, etc. The system shown in FIG. 3A and FIG. 3B comprises three encapsulation devices (110).

The stacked devices (110) of the system (100) are connected together, e.g., to prevent sliding. In some embodiments, the devices (110) are sutured together (see FIG. 3A). In some embodiments, the devices (110) are connected via other means (e.g., welding but with unwelded spaces between them so as to avoid blocking blood vessels). FIG. 3A shows sutures (170) or spacers linking the different devices (110). FIG. 3A also shows the vasculature (210) around and within the system (100). Note the vasculature may encompass the entire system (100), which is not shown in FIG. 3A or FIG. 3B. A seal (172) is shown surrounding the device in FIG. 3B.

The connecting components space the encapsulation devices apart to allow vasculature to grow between them.

The stacked devices (110) of the system (100) are configured (e.g., spaced a distance apart) to allow for vascularization between the individual devices.

As previously discussed, the encapsulation devices (110) may be constructed in a variety of sizes and with a variety of different lumen volumes. In some embodiments, the devices (110) in the system (100) are uniform in length and/or lumen volume. In some embodiments, one or more of the devices (110) in the system (100) has a different length and/or lumen volume. For example, a first device (110) may be about 4.5 cm in length and a second device may be about 2 cm in length.

In some embodiments, the systems or devices of the present invention feature a sealant and/or a scaffold disposed between the individual encapsulation devices. Scaffold or sealant materials may include but are not limited to a gel, e.g., fibrin (e.g., fibrin sealant). The scaffold or sealant allows for vascularization to occur.

### Incorporation of Oxygen Delivery

Without wishing to limit the present invention to any theory or mechanism, one of ordinary skill in the art may believe that adding oxygen to the system may inhibit vessel growth, e.g., the opposite of what occurs in hypoxic situations that stimulate vessel growth. However, the system of the present invention may be used with oxygen (or air) delivery. In some embodiments, an oxygen delivery system is integrated into the system, e.g., integrated within the individual stacked encapsulation devices. The amount of oxygen supplied to the systems (if applicable), may vary, e.g., low oxygen may be supplied, atmospheric oxygen levels may be supplied, higher oxygen levels may be supplied, etc.

The present invention is not limited to systems that feature oxygen delivery. In some embodiments, exogenous oxygen is not incorporated into the system.

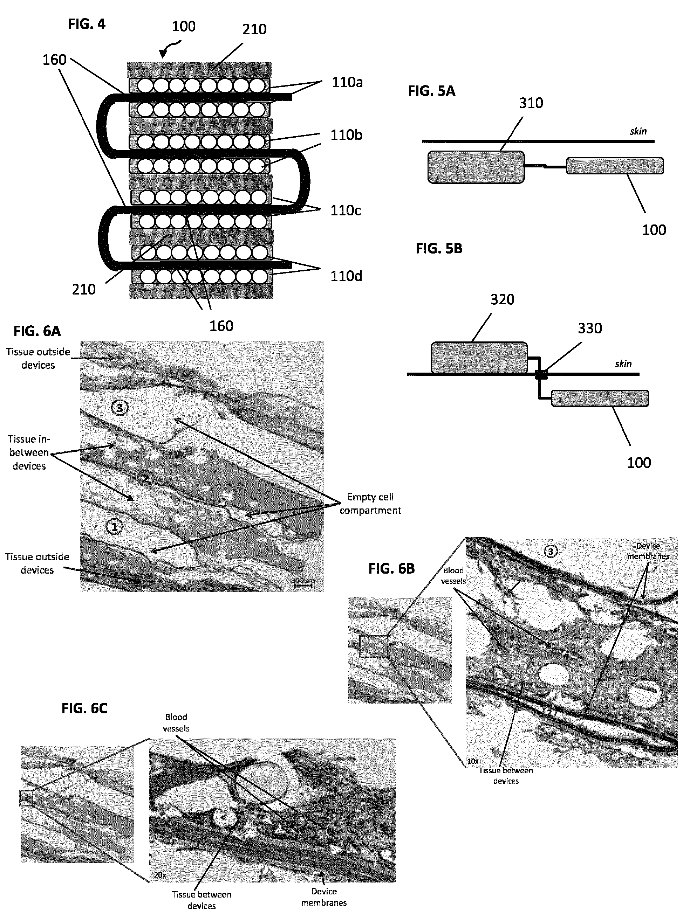

According to the present invention, the system (100) of the present invention comprises a channel, such as a gas channel (160) for delivery of the gas or other fluids (e.g., with nutrients) to the cells in an encapsulation device (e.g., to multiple lumens inside a single encapsulation device). As shown in FIG. 4, a gas channel may fluidly connect two or more individual encapsulation devices. The gas channel (160) in FIG. 4 is disposed in between two lumens of a first encapsulation device and extends out of the device and into a second encapsulation device in between its two lumens.

FIG. 4 shows a system (100) comprising a first encapsulation device (110a) with two lumens separated by a gas chamber (160) stacked atop a second encapsulation device (110b) with two lumens separated by a gas chamber (160) stacked atop a third encapsulation device (110c) with two lumens separated by a gas chamber (160) stacked atop a fourth encapsulation device (110d) with two lumens separated by a gas chamber (160). The gas chambers (160) are fluidly connected such that gas flows through the first device (110a) to the second device (110b) to the third device (110c) to the fourth device (110d) and out of the fourth device (110d). Vascularization (210) is present around and between the four devices (110).

In some embodiments, the devices of the systems of the present invention are temporarily oxygenated. For example, in some embodiments, oxygen is temporarily delivered initially (e.g., initially upon implantation) until the system is adequately vascularized. In some embodiments, oxygen may be temporarily delivered and/or oxygen levels may be variable. For example, in some embodiments, a cell type is used that benefits from a high oxygen level. In some embodiments, a cell type is used that benefits from a low oxygen level (e.g., 15% or lower). In some embodiments, an oxygen level of about 21% oxygen (e.g., 20-22%) is used, e.g., air may be used. In some embodiments, an oxygen level from 15-22% is used. In some embodiments, an oxygen level from 10-15% is used. In some embodiments, an oxygen level from 5-10% is used. In some embodiments, an oxygen level from 0-5% is used. In some embodiments, a particular oxygen level is used initially and then the oxygen level is increased or decreased at a later time. In some embodiments, oxygen is turned on and then off. In some embodiments, oxygen is turned off and then on. In some embodiments, oxygen is turned on and off in a cycle for a period of time or indefinitely. In some embodiments, oxygen level is tailored to the application to help modulate the local immune system by providing temporary oxygen. In some embodiments, oxygen levels are tailed to when vascularization occurs. In some embodiments, immature cells are transplanted, and low oxygen levels may be used initially; as the cells mature (e.g., after a particular time, e.g., 4-6 weeks), higher oxygen levels may be provided.

Referring to FIG. 5A and FIG. 5B, oxygen may be delivered to the systems via several different mechanisms. For example, in FIG. 5A, the system (100) is operatively and fluidly connected to an implantable oxygen generator (310). Tubing delivers gas to the gas channel (160) of the system (100). Implantable oxygen generators are well known to one of ordinary skill in the art. For example, the implantable oxygen generator may feature an electrochemical oxygen generation mechanism (e.g., using electricity to break down water to oxygen hydrogen), a chemical mechanism, or other mechanism. In FIG. 5B, the system (100) is operatively and fluidly connected to a wearable oxygen generator (320) or pump via tubing. A special device (330) may be implanted into the skin to help prevent infection.

In some embodiments, the oxygen is delivered via a carrier media like hemoglobin or fluorinated microbubbles. The present invention is not limited to the aforementioned systems or materials.

In some embodiments, there is a contiguous gas supply through each of the devices (110).

### Sensors

In some embodiments, the system features one or more sensors disposed on or in one or more places of the system (100). For example, in some embodiments, a sensor is disposed in the gas channel (160). In some embodiments, a sensor is disposed in a lumen of a device (110). In some embodiments, a sensor is disposed on the outer surface of a device (110). Sensors may include but are not limited to oxygen sensors, glucose sensors, lactate sensors, or other appropriate sensors. In some embodiments, the system comprises a means (e.g., a sensor) for determining when the cells are dead (e.g., via oxygen sensors, etc.).

Without wishing to limit the present invention to any theory or mechanism, cells are likely dead if there is generally no difference in oxygen levels inside and outside the device. Typically there is a difference (a gradient) in oxygen levels between the inside and outside of the device because oxygen is being consumed by live cells. Thus, no difference would be indicative of no oxygen consumption, thus the cells are likely dead. A bigger difference (gradient) in oxygen levels between the inside and outside of the device would indicate there are more viable cells. A user may determine how many cells are dying by determining the change in oxygen gradient.

As previously discussed, the systems or devices may be implanted at any appropriate site, including but not limited to an arm location, a leg location, a torso location, etc.

### Example 1

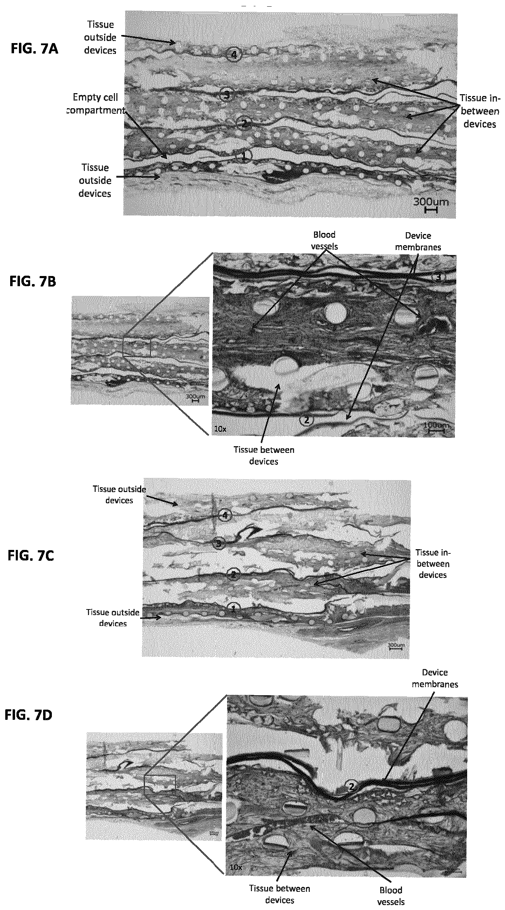

Three 40uL 1-chamber encapsulation devices were stitched together at four spots (top, bottom, and sides) using 3-0 silk suture. Devices were placed on top of each other with the loading ports facing opposite directions (see FIG. 3A). A system with the three empty 40uL 1-chamber encapsulation devices was implanted subcutaneously into one althymic nude rat. The system was implanted for 24 days. On day 24, the system was explanted and processed for histology. The system was paraffin embedded, sectioned, and stained with H and E (Hematoxylin, nuclei in purple, and Eosin, cytoplasm in pink). Images were collected using Keyence BZ-X700 Fluorescence microscope. Resulting images are shown in FIG. 6, FIG. 7, and FIG. 8.

### Example 2

Four 4.5uL 1-chamber encapsulation devices and four 20uL 1-chamber were stitched together at four spots (top, bottom, and sides) using 3-0 silk suture. Devices were placed on top of each other with the loading ports facing opposite directions (see FIG. 3A for example). The system with four empty 4.5uL 1-chamber encapsulation devices was implanted subcutaneously into one althymic nude rat and the system with four 20uL 1-chamber encapsulation devices was implanted subcutaneous into another althymic nude rat. The 4.5uL system was transplanted for 45 days then explanted. The 20uL system was transplanted for 60 days and then explanted. Both systems were processed for histology. The systems were paraffin embedded, sectioned, and stained with H and E (Hematoxylin, nuclei in purple, and Eosin, cytoplasm in pink). Images were collected using Keyence BZ-X700 Fluorescence microscope. Resulting images are shown in FIG. 9, FIG. 10, FIG. 11, and FIG. 12.

Various modifications of the invention, in addition to those described herein, will be apparent to those skilled in the art from the foregoing description. Such modifications are also intended to fall within the scope of the appended claims.

Although there has been shown and described the preferred embodiment of the present invention, it will be readily apparent to those skilled in the art that modifications may be made thereto which do not exceed the scope of the appended claims. Therefore, the scope of the invention is only to be limited by the following claims. Reference numbers recited in the claims are exemplary and for ease of review by the patent office only, and are not limiting in any way. In some embodiments, the figures presented in this patent application are drawn to scale, including the angles, ratios of dimensions, etc. In some embodiments, the figures are representative only and the claims are not limited by the dimensions of the figures. In some embodiments, descriptions of the inventions described herein using the phrase "comprising" includes embodiments that could be described as "consisting of", and as such the written description requirement for claiming one or more embodiments of the present invention using the phrase "consisting of" is met.

The reference numbers recited in the below claims are solely for ease of examination of this patent application, and are exemplary, and are not intended in any way to limit the scope of the claims to the particular features having the corresponding reference numbers in the drawings.

## Claims

1. A system (100) comprising at least a first encapsulation device (110a) stacked on a second encapsulation device (110b) and connected by a connecting component to space the first and second encapsulation devices apart, wherein the connecting component allows vasculature to grow between the two encapsulation devices, wherein the first encapsulation device (110a) comprises a lumen for holding cells, a vascularization membrane and a channel (160) for delivery of gas or other fluids to cells in the first encapsulation device, wherein the second encapsulation device (110b) comprises a lumen for holding cells, a vascularization membrane and a channel (160) for delivery of gas or other fluids to cells in the second encapsulation device.

2. The system of claim 1, wherein vasculature surrounds at least a portion of the system and is disposed in at least a portion of a space between the first encapsulation device and the second encapsulation device.

3. The system of claim 1 or claim 2, wherein the connecting component comprises a suture (170) or a weld.

4. The system of any of claims 1-3 wherein the first encapsulation device further comprises an immunoisolation membrane (130) in between the lumen and the vascularization membrane.

5. The system of any of claims 1**-**4 wherein the second encapsulation device further comprises an immunoisolation membrane (130) in between the lumen and the vascularization membrane.

6. The system of any of claims 1**-**5 wherein the first encapsulation device and the second encapsulation device each comprise two lumens separated by a gas channel (160).

7. The system of claim 6, wherein the gas channel (160) of the first encapsulation device is fluidly connected to the gas channel (160) of the second encapsulation device.

8. The system of claim 7, wherein the channels (160) are fluidly connected so that there is a contiguous gas supply through each of the devices (110a, 110b).

9. The system of any of claims 1-8 further comprising cells disposed in the lumen of the first encapsulation device and the second encapsulation device.

10. The system of claim 9, wherein the cells comprise spheroids that can produce and release a therapeutic agent.

11. The system of any of claims 1-10, operatively connected to an oxygen generator (310, 320).

12. The system of any of claims 1-11, wherein a sensor is disposed in a gas channel of the system.

## Patentansprüche

1. System (100), umfassend wenigstens eine erste Verkapselungsvorrichtung (110a), die auf einer zweiten Verkapselungsvorrichtung (110b) gestapelt und durch eine Verbindungskomponente verbunden ist, um die erste und die zweite Verkapselungsvorrichtung voneinander zu beabstanden, wobei die Verbindungskomponente das Wachstum von Blutgefäßen zwischen den zwei Verkapselungsvorrichtungen ermöglicht, wobei die erste Verkapselungsvorrichtung (110a) ein Lumen zum Halten von Zellen, eine Vaskularisierungsmembran und einen Kanal (160) für die Abgabe von Gas oder anderen Flüssigkeiten an Zellen in der ersten Verkapselungsvorrichtung umfasst, wobei die zweite Verkapselungsvorrichtung (110b) ein Lumen zum Halten von Zellen, eine Vaskularisierungsmembran und einen Kanal (160) für die Abgabe von Gas oder anderen Flüssigkeiten an Zellen in der zweiten Verkapselungsvorrichtung umfasst.

2. System nach Anspruch 1, wobei die Blutgefäße wenigstens einen Abschnitt des Systems umgeben und in wenigstens einem Abschnitt eines Raums zwischen der ersten Verkapselungsvorrichtung und der zweiten Verkapselungsvorrichtung angeordnet sind.

3. System nach Anspruch 1 oder 2, wobei die Verbindungskomponente eine Naht (170) oder eine Schweißnaht umfasst.

4. System nach einem der Ansprüche 1 - 3, wobei die erste Verkapselungsvorrichtung ferner eine Immunisolationsmembran (130) zwischen dem Lumen und der Vaskularisationsmembran umfasst.

5. System nach einem der Ansprüche 1 - 4, wobei die zweite Verkapselungsvorrichtung ferner eine Immunisolationsmembran (130) zwischen dem Lumen und der Vaskularisationsmembran umfasst.

6. System nach einem der Ansprüche 1 - 5, wobei die erste Verkapselungsvorrichtung und die zweite Verkapselungsvorrichtung jeweils zwei durch einen Gaskanal (160) getrennte Lumen umfassen.

7. System nach Anspruch 6, wobei der Gaskanal (160) der ersten Verkapselungsvorrichtung mit dem Gaskanal (160) der zweiten Verkapselungsvorrichtung in Fluidverbindung steht.

8. System nach Anspruch 7, wobei die Kanäle (160) fluidisch verbunden sind, sodass es eine kontinuierliche Gaszufuhr durch jede der Vorrichtungen (110a, 110b) gibt.

9. System nach einem der Ansprüche 1 - 8, ferner umfassend Zellen, die in dem Lumen der ersten Verkapselungsvorrichtung und der zweiten Verkapselungsvorrichtung angeordnet sind.

10. System nach Anspruch 9, wobei die Zellen Sphäroide umfassen, die einen therapeutisches Mittel produzieren und freisetzen können.

11. System nach einem der Ansprüche 1 - 10, das mit einem Sauerstoffgenerator (310, 320) wirkverbunden ist.

12. System nach einem der Ansprüche 1 - 11, wobei ein Sensor in einem Gaskanal des Systems angeordnet ist.

## Revendications

1. Système (100) comprenant au moins un premier dispositif d'encapsulation (110a) et un deuxième dispositif d'encapsulation (110b) empilés l'un sur l'autre et reliés par un composant de raccord destiné à espacer les premier et deuxième dispositifs d'encapsulation, ledit composant de raccord permettant à une vasculature de croître entre les deux dispositifs d'encapsulation, ledit premier dispositif d'encapsulation (110a) comprenant une lumière destinée à contenir des cellules, une membrane de vascularisation et un canal (160) permettant l'apport de gaz ou d'autres fluides auxdites cellules du premier dispositif d'encapsulation, ledit deuxième dispositif d'encapsulation (110b) comprenant une lumière destinée à contenir des cellules, une membrane de vascularisation et un canal (160) permettant l'apport de gaz ou d'autres fluides aux cellules du deuxième dispositif d'encapsulation.

2. Système selon la revendication 1, dans lequel la vasculature entoure au moins une partie du système et est située dans au moins une partie d'un espace existant entre le premier dispositif d'encapsulation et le deuxième dispositif d'encapsulation.

3. Système selon la revendication 1 ou la revendication 2, dans lequel le composant de raccord comprend une suture (170) ou une soudure.

4. Système selon l'une quelconque des revendications 1 à 3, dans lequel le premier dispositif d'encapsulation comprend en outre une membrane d'immuno-isolation (130) entre la lumière et la membrane de vascularisation.

5. Système selon l'une quelconque des revendications 1 à 4, dans lequel le deuxième dispositif d'encapsulation comprend en outre une membrane d'immuno-isolation (130) entre la lumière et la membrane de vascularisation.

6. Système selon l'une quelconque des revendications 1 à 5, dans lequel le premier dispositif d'encapsulation et le deuxième dispositif d'encapsulation comprennent chacun deux lumières séparées par un canal de gaz (160).

7. Système selon la revendication 6, dans lequel le canal de gaz (160) du premier dispositif d'encapsulation est relié fluidiquement au canal de gaz (160) du deuxième dispositif d'encapsulation.

8. Système selon la revendication 7, dans lequel les canaux (160) sont reliés fluidiquement de telle façon qu'il existe une alimentation en gaz contiguë à travers chacun des dispositifs (110a, 110b).

9. Système selon l'une quelconque des revendications 1 à 8, comprenant en outre des cellules situées dans la lumière du premier dispositif d'encapsulation et du deuxième dispositif d'encapsulation.

10. Système selon la revendication 9, dans lequel les cellules comprennent des sphérules susceptibles de produire et de libérer un agent thérapeutique.

11. Système selon l'une quelconque des revendications 1 à 10, relié de manière opérationnelle à un générateur d'oxygène (310, 320).

12. Système selon l'une quelconque des revendications 1 à 11, dans lequel un capteur est disposé dans un canal de gaz du système.
